# EUROPÄISCHE PATENTANMELDUNG

(11) **EP 2 044 967 A1**
(43) Veröffentlichungstag der Anmeldung: **08.04.2009**
(21) Anmeldenummer: 07019265.3
(22) Anmeldetag: 01.10.2007
(51) Int. Cl.: A61M 15/00, B05B 1/00, A61M 11/06

(54) **Zerstäuber**

(71) Anmelder: Boehringer Ingelheim Pharma GmbH & Co. KG, 55216 Ingelheim (DE)
(72) Erfinder: Die Erfindernennung liegt noch nicht vor

(57) **Zusammenfassung**

Es wird ein Zerstäuber (1) zur Zerstäubung einer Arzneimittelzubereitung (2) zur medizinischen Aerosol-Therapie vorgeschlagen. Der Zerstäuber weist eine Austragsdüse (12) für die Arzneimittelzubereitung auf. Stromauf der Austragsdüse sind ein Vorfilter (27) und ein Feinstfilter (12c) angeordnet. Stromab der Austragsdüse ist ein Halteelement (31) zur Halterung der Austragsdüse angeordnet. Weiter ist eine Zähleinrichtung (41) mit einer Gewindespindel (42) und einem zugeordneten Reiter (43) sowie mit Zählmarkierungen (44) vorgesehen. Die Arzneimittelzubereitung enthält mindestens ein Tiotropiumsalz als Wirkstoff.

## Beschreibung

Die vorliegende Erfindung betrifft einen Zerstäuber gemäß dem Oberbegriff einer der unabhängigen Patentansprüche.

Ausgangspunkt der vorliegenden Erfindung ist ein Zerstäuber in Form eines Inhalators, wie im Grundprinzip in der WO 91/14468 A1 und in konkreter Ausgestaltung in der WO 97/12687 A1 (Fig. 6a, 6b) dargestellt. Der Zerstäuber weist als Reservoir für eine zu zerstäubende Arzneimittelzubereitung einen einsetzbaren, starren Behälter mit einem Innenbeutel mit der Arzneimittelzubereitung und einen Druckerzeuger mit einer Antriebsfeder zur Förderung und Zerstäubung der Arzneimittelzubereitung auf.

Der vorliegenden Erfindung liegt die Aufgabe zugrunde, einen neuen bzw. verbesserten Zerstäuber anzugeben.

Die obige Aufgabe wird durch einen Zerstäuber gemäß einem der unabhängigen Patentansprüche gelöst, Vorteilhafte Weiterbildungen sind Gegenstand der Unteransprüche.

Ein Aspekt der vorliegenden Erfindung liegt darin, einen Vorfilter stromauf der Austragsdüse anzuordnen. So können eventuell vorhandene Teilchen herausgefiltert werden, die die Austragsdüse verstopfen oder verlegen könnten. Dies ist einer sicheren Funktion des Zerstäubers und optimalen Zerstäubung zuträglich.

Ein anderer Aspekt der vorliegenden Erfindung sieht vor, daß der Zerstäuber ein vorzugsweise scheibenartiges und/oder relativ weiches Halteelement aufweist, das stromab der Austragsdüse angeordnet ist und diese hält. Dies ermöglicht insbesondere bei einer sehr kleinen bzw. feinen Austragsdüse eine einfache Montage und/oder sichere Halterung. Weiter ist dies einer sicheren Funktion des Zerstäubers und optimalen Zerstäubung zuträglich.

Gemäß einem weiteren Aspekt der vorliegenden Erfindung weist der Zerstäuber ein erstes Gehäuseteil und ein dazu drehbares Innenteil auf. Das Innenteil ist vorzugsweise über ein zweites Gehäuseteil zum Betätigen bzw. Spannen des Zerstäubers, insbesondere Spannen einer Antriebsfeder des Zerstäubers, drehbar. Die Relativdrehung des Innenteils zum ersten Gehäuseteil treibt eine Zähleinrichtung des Zerstäubers an. Dies gestattet einen sehr einfachen und funktionssicheren Aufbau des Zerstäubers.

Ein anderer Aspekt der vorliegenden Erfindung liegt darin, daß die Zähleinrichtung eine Gewindespindel mit einem zugeordneten, durch Drehen der Gewindespindel insbesondere linear bewegbaren Reiter sowie dem Reiter zugeordnete Zählmarkierungen aufweist. Dies gestattet wiederum einen sehr einfachen und funktionssicheren Aufbau.

Noch ein anderer Aspekt der vorliegenden Erfindung liegt darin, daß der Zerstäuber ein erstes Gehäuseteil und ein zum Spannen des Zerstäubers relativ zum ersten Gehäuseteil um jeweils 180° drehbares Teil aufweist, wobei diese Relativdrehung um 180° jeweils eine Gewindespindel der Zähleinrichtung weiterdreht, insbesondere jeweils um eine halbe Umdrehung. Dies gestattet einen besonders einfachen und funktionssicheren Aufbau.

Ein weiterer Aspekt der vorliegenden Erfindung liegt darin, den Zerstäuber derart auszubilden, daß der Zerstäuber zur Ausbringung einer Dosis der Arzneimittelzubereitung zweimal hintereinander betätigt, nämlich zweimal hintereinander gespannt und ausgelöst werden muß und diese zweimalige Betätigung nur als eine Betätigung oder eine abgegebene Dosis des Zerstäubers von der Zähleinrichtung erfaßt oder gezählt wird. Dies gestattet eine effektive und funktionssichere Ausbringung der Arzneimittelzubereitung und des genannten Wirkstoffs, auch bei einer größeren zu zerstäubenden Menge.

Ein besonders bevorzugter Aspekt der vorliegenden Erfindung liegt darin, daß der Zerstäuber eine Arzneimittelzubereitung mit einem Tiotropiumsalz oder mehreren Tiotropiumsalzen als Wirkstoff enthält. Insbesondere wird die Arzneimittelzubereitung als Flüssigkeit treibgasfrei zerstäubt. Dies gestattet eine sehr effektive und funktionssichere Ausbringung der Arzneimittelzubereitung und des genannten Wirkstoffs.

Ein weiterer Aspekt der vorliegenden Erfindung sieht vor, daß der Zerstäuber eine Einrichtung zur zwangsweisen Belüftung eines Behälters aufweist, wobei in dem Behälter ein flexibler, bei der Entnahme kollabierender Innenbeutel mit der zu zerstäubenden Arzneimittelzubereitung angeordnet ist. Dies ist der Funktionssicherheit und Dosiergenauigkeit des Zerstäubers und optimalen Zerstäubung sowie einer langen Lagerfähigkeit zuträglich.

Die einzelnen Aspekte der vorliegenden Erfindung können unabhängig voneinander und/oder in einer beliebigen Kombination miteinander verwirklicht werden.

Weitere Vorteile, Merkmale, Eigenschaften und Aspekte der vorliegenden Erfindung ergeben sich aus den Ansprüchen und der folgenden Beschreibung einer bevorzugten Ausführungsform anhand der Zeichnung. Es zeigt:
- Fig. 1: einen schematischen Schnitt eines Zerstäubers im ungespannten Zustand mit einem Behälter, aber ohne Mundstückabdeckung;
- Fig. 2: einen schematischen, um 90° gegenüber Fig. 1 gedrehten Schnitt des Zerstäubers im gespannten Zustand;
- Fig. 3: einen schematischen, zu Fig. 2 korrespondierenden Schnitt des Zerstäubers im gespannten Zustand, jedoch ohne Behälter, aber mit Mundstückabdeckung;
- Fig. 4: eine schematische, explosionsartige Darstellung der Einzelteile des Zerstäubers;
- Fig. 5: eine ausschnittsweise explosionsartige Darstellung von einzelnen Bauteilen mit einer Austragsdüse des Zerstäubers;
- Fig. 6: eine schematische Draufsicht eines die Düsenstruktur zeigenden Teils der Austragsdüse; und
- Fig. 7: eine schematische Seitenansicht des Zerstäubers.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

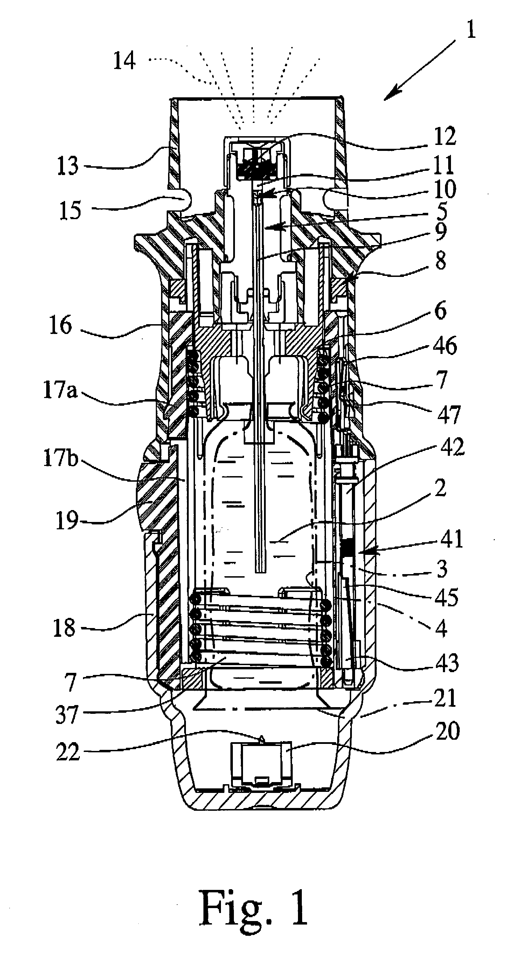

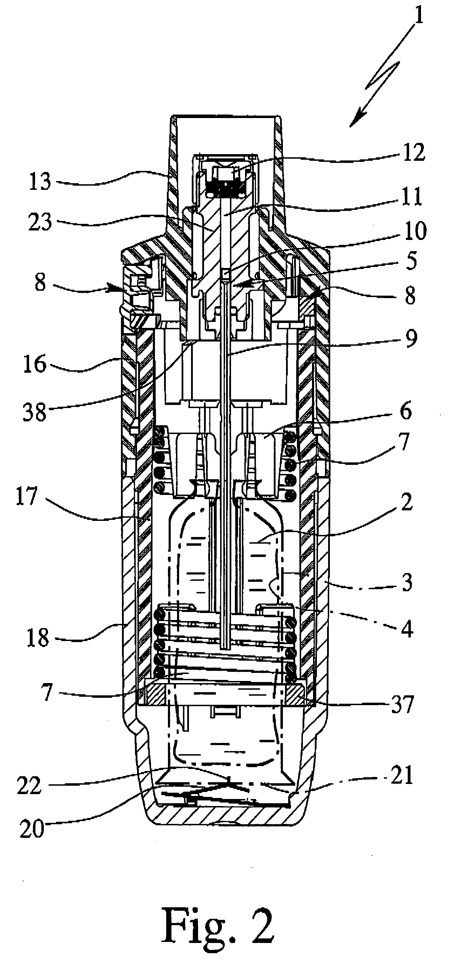

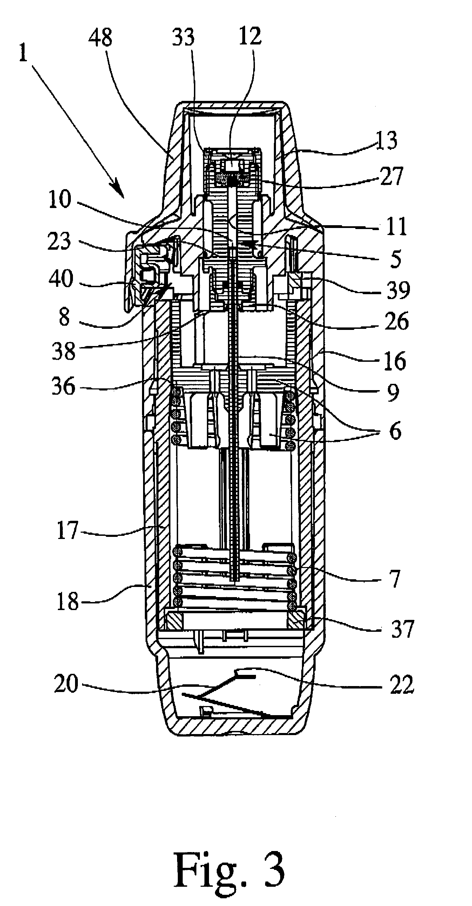

Fig. 1 bis 3 zeigen einen vorschlagsgemäßen Zerstäuber 1 zur Zerstäubung einer Arzneimittelzubereitung 2 in einer schematischen Darstellung im ungespannten Zustand (Fig. 1) und gespannten Zustand (Fig. 2 und Fig. 3). Der Zerstäuber 1 ist insbesondere als tragbarer Inhalator ausgebildet und arbeitet vorzugsweise ohne Treibgas.

Fig. 1 und 2 zeigen den Zerstäuber 1 mit einem Behälter 3 mit der Arzneimittelzubereitung 2. Fig. 3 zeigt den Zerstäuber 1 ohne Behälter 3.

Bei Zerstäubung der Arzneimittelzubereitung 2, vorzugsweise einer Flüssigkeit, wird ein lungengängiges Aerosol 14 (Fig. 1) gebildet, das von einem nicht dargestellten Benutzer bzw. Patienten eingeatmet bzw. inhaliert werden kann. Üblicherweise erfolgt das Inhalieren wenigstens einmal täglich, insbesondere mehrmals täglich, vorzugsweise in vorbestimmten Zeitabständen, insbesondere in Abhängigkeit von der Erkrankung des Patienten.

Der Zerstäuber 1 weist den vorzugsweise einsetzbaren und ggf. wechselbaren Behälter 3 mit der Arzneimittelzubereitung 2 auf. Der Behälter 3 bildet also ein Reservoir für die zu zerstäubende Arzneimittelzubereitung 2. Vorzugsweise enthält der Behälter 3 eine ausreichende Menge an Arzneimittelzubereitung 2 bzw. Wirkstoff für mehrere Dosen des Arzneimittelzubereitung 2, also mehrere Zerstäubungen oder Anwendungen zu ermöglichen. Ein typischer Behälter 3, wie in der WO 96/06011 A1 offenbart, nimmt ein Volumen von ca. 2 bis 10 ml auf. Hinsichtlich des bevorzugten Aufbaus des Behälters 3 wird ergänzend auf die WO 00/49988 A2 verwiesen.

Der Behälter 3 ist vorzugsweise im wesentlichen zylindrisch bzw. kartuschenartig ausgebildet und von unten, nach Öffnen des Zerstäubers 1, in diesen einsetzbar und ggf. wechselbar. Er ist vorzugsweise starr ausgebildet, insbesondere wobei das Arzneimittelzubereitung 2 in einem kollabierbaren Beutel 4 im Behälter 3 aufgenommen ist.

Der Zerstäuber 1 weist ferner eine Fördereinrichtung, insbesondere einen Druckerzeuger 5, zur Förderung und Zerstäubung der Arzneimittelzubereitung 2, insbesondere jeweils in einer vorbestimmten, ggf. einstellbaren Dosiermenge auf.

Der Zerstäuber 1 bzw. Druckerzeuger 5 weist insbesondere eine Halterung 6 für den Behälter 3, eine zugeordnete, nur teilweise dargestellte Antriebsfeder 7 vorzugsweise mit einem zugeordneten, zur Entsperrung manuell betätigbaren Sperrelement 8, ein vorzugsweise als Kapillare ausgebildetes Förderrohr 9 mit einem optionalen Ventil, insbesondere Rückschlagventil 10, eine Druckkammer 11 und/oder eine Austragsdüse 12 insbesondere im Bereich eines Mundstücks 13 auf.

Der Behälter 3 wird über die Halterung 6, insbesondere klemmend oder rastend, so in dem Zerstäuber 1 fixiert, daß das Förderrohr 9 in den Behälter 3 eintaucht. Die Halterung 6 kann dabei derart ausgebildet sein, daß der Behälter 3 ausgetauscht werden kann.

Beim axialen Spannen der Antriebsfeder 7 wird die Halterung 6 mit dem Behälter 3 und dem Förderrohr 9 bei den Darstellungen nach unten bewegt und die Arzneimittelzubereitung 2 - genauer gesagt die nächste Dosis - aus dem Behälter 3 in die Druckkammer 11 des Druckerzeugers 5 über das Rückschlagventil 10 gesaugt.

Beim anschließenden Entspannen nach Betätigung des Sperrelements 8 wird die Arzneimittelzubereitung 2 in der Druckkammer 11 unter Druck gesetzt, indem das Förderrohr 9 bei nun geschlossenem Rückschlagventil 10 durch Entspannen der Antriebsfeder 7 wieder nach oben bewegt wird und nun als Druckstempel wirkt. Dieser Druck treibt die Arzneimittelzubereitung 2 durch die Austragsdüse 12 aus, wobei es in das vorzugsweise lungengängige Aerosol 14 zerstäubt wird, wie in Fig. 1 angedeutet.

Der nicht dargestellte Benutzer bzw. Patient kann das Aerosol 14 inhalieren, wobei vorzugsweise Zuluft über mindestens eine Zuluftöffnung 15 in das Mundstück 13 saugbar ist.

Während des Zerstäubungsvorgangs wird der Behälter 3 von der Antriebsfeder 7 wieder in seine Ausgangslage zurückbewegt.

Der Behälter 3 führt also eine Hubbewegung während des Spannvorgangs und während des Zerstäubungsvorgangs aus.

Der Zerstäuber 1 weist insbesondere ein erstes Gehäuseteil (Oberteil) 16 und ein demgegenüber drehbares Innenteil 17 (Fig. 2) mit einem oberen Teil 17a und einem unteren Teil 17b (Fig. 1) auf, wobei an dem Innenteil 17 ein insbesondere manuell betätigbares oder drehbares, zweites Gehäuseteil (Unterteil) 18 vorzugsweise mittels eines Sicherheitsverschlusses bzw. Halteelementes 19 lösbar befestigt, insbesondere aufgesteckt, ist. Insbesondere ist der Sicherheitsverschluß bzw. das Halteelement 19 derart ausgebildet, daß ein versehentliches Öffnen des Zerstäubers 1 bzw. Abziehen des zweiten Gehäuseteils 18 ausgeschlossen ist. Insbesondere muß zum Lösen des zweiten Gehäuseteils 18 das Halteelement 19 gegen Federkraft eingedrückt werden. Zum Einsetzen und/oder Auswechseln des Behälters 3 ist das zweite Gehäuseteil 18 vom Zerstäuber 1 lösbar. Das zweite Gehäuseteil 18 bildet vorzugsweise ein kappenartiges Gehäuseunterteil und/oder um- bzw. übergreift einen unteren freien Endbereich des Behälters 3.

Das zweite Gehäuseteil 18 kann relativ zum ersten Gehäuseteil 16 gedreht werden, wobei das Innenteil 17 mitgedreht wird. Dadurch wird die Antriebsfeder 7 über ein, nicht im einzelnen dargestelltes, auf die Halterung 6 wirkendes Getriebe in axialer Richtung gespannt. Mit dem Spannen wird der Behälter 3 axial nach unten bzw. mit seinem Endbereich (weiter) in das zweite Gehäuseteil 18 bzw. zu dessen stirnseitigem Ende hin bewegt, bis der Behälter 3 eine in Fig. 2 angedeutete Endlage einnimmt. In diesem Zustand ist die Antriebsfeder 7 gespannt.

Der Zerstäuber 1 weist vorzugsweise eine Einrichtung zur zwangsweisen Belüftung des Behälters 3 auf.

Beim erstmaligen Spannen erfolgt vorzugsweise ein bodenseitiges Anstechen bzw. Öffnen des Behälters 3. Insbesondere kommt eine axial wirkende, im Gehäuseteil 18 angeordnete Feder 20 am Behälterboden 21 zur Auflage, die mit einem Anstechelement 22 den Behälter 3 bzw. eine bodenseitige, insbesondere gasdichte Versiegelung bei der erstmaligen Anlage zur Belüftung ansticht.

Die Einrichtung zur zwangsweisen Belüftung ist hier also durch das Anstechelement 22 gebildet, das von der Feder 20 gehalten oder gebildet ist. Jedoch sind auch andere konstruktive Lösungen möglich.

Es ist anzumerken, daß bei dem Anstechen zur Belüftung lediglich die Außenhülle des Behälters 3 geöffnet wird. Der Beutel 4 mit der Arrzneimittelzubereitung 2 bleibt unbeschädigt. Bei der Entnahme der Arzneimittelformulierung 2 aus dem Beutel 4 über das Förderrohr 9 kollabiert der flexible Beutel 4. Zum Druckausgleich kann die Umgebungsluft über die Belüftungs- bzw. Anstechöffnung in den Behälter 3 nachströmen.

Zur Benutzung des Zerstäubers 1 muß zunächst der Behälter 3 eingesetzt werden. Dies erfolgt vorzugsweise dadurch, daß das zweite Gehäuseteil 18 entfernt bzw. abgezogen wird. Anschließend wird der Behälter 3 in das Innenteil 17 axial eingeführt bzw. eingeschoben. Hierbei erfolgt ein kopfseitiges Öffnen bzw. Anschließen des Behälters 3. Dies erfolgt durch das Förderelement, also das Förderrohr 9, das eine vorzugsweise vorgesehene, kopfseitige Versiegelung des Behälters 3 durchsticht und anschließend durch ein kopfseitiges Septum des Behälters 3 hindurch in das Innere des Beutels 4 eingeführt wird. So wird die fluidische Verbindung zwischen dem Behälter 3 - genauer gesagt zwischen dem Beutel 4 im Behälter 3 - über das Förderrohr 9 zum Druckerzeuger 5 bzw. zur Druckkammer 11 hergestellt.

Anschließend wird das zweite Gehäuseteils 18 wieder aufgeschoben. Nun kann das erstmalige Spannen des Zerstäubers 1 erfolgen. Hierbei wird dann der Behälter 3 durch das Anstechelement 22 bodenseitig angestochen, also zwangsweise belüftet, wir bereits erläutert.

Vor der erstmaligen Benutzung erfolgt nach dem Einsetzen bzw. fluidischen Anschließen des Behälters 3 ein vorzugsweise mehrmaliges Spannen und Auslösen des Zerstäubers 1. Durch dieses sogenannte Primen wird im Förderrohr 9 und im Druckerzeuger 5 bis zur Austragsdüse 12 eventuell vorhandene Luft von der Arzneimittelzubereitung 2 verdrängt. Anschließend ist der Zerstäuber 1 zur Inhalation bereit.

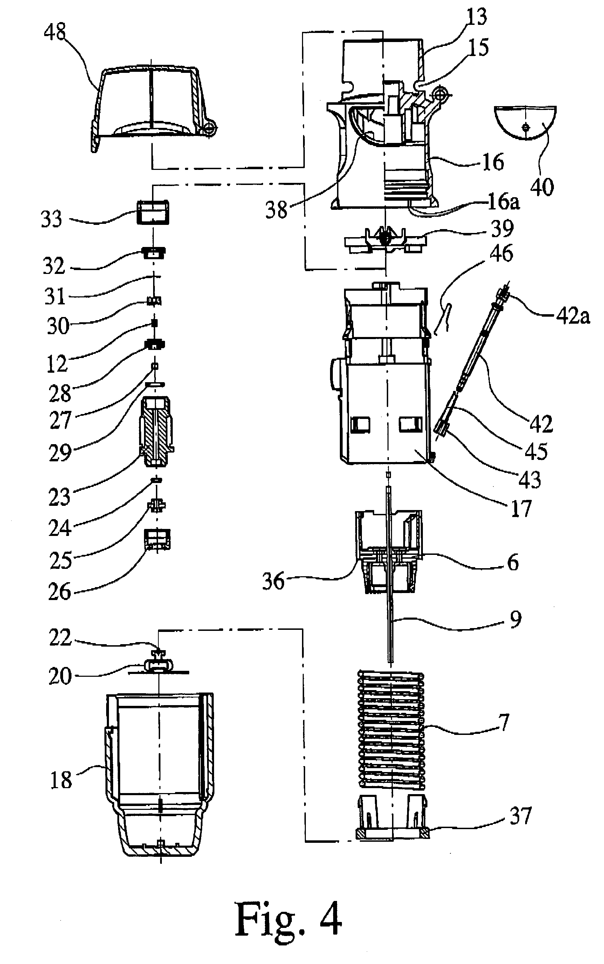

Fig. 4 zeigt in einer schematischen, explosionsartigen Darstellung die Einzelteile des Zerstäubers 1.

Der Druckerzeuger 5 weist ein insbesondere rohrförmiges Zentralteil 23 auf, das mit einer Längsbohrung versehen ist, die die Druckkammer 11 bildet. Das Förderrohr 9 ragt vorzugsweise abgedichtet in die Druckkammer 11. Hierzu ist vorzugsweise eine Dichtung 24, insbesondere ein O-Ring, von einem Stützring 25 und einer Überwurfmutter 26 in einer entsprechenden Ausnehmung am behälterseitigen bzw. zuführungsseitigen Ende des Zentralteils 23 gehalten. Das Förderrohr 9 erstreckt sich im montierten Zustand durch die Dichtung 24 hindurch und wird durch diese außenseitig bzw. radial abgedichtet.

Hinsichtlich des Einbaus und der Ausgestaltung der Dichtung 24 wird ergänzend auf die WO 2004/053362 A1 verwiesen.

Am anderen bzw. auslaßseitigen Ende des Zentralteils 23 wird die Austragsdüse 12 montiert. Zwischen der Austragsdüse 12 und der Druckkammer 11 bzw. stromauf der Austragsdüse 12 ist vorzugsweise ein Vorfilter 27 angeordnet.

Der Vorfilter 27 dient insbesondere einem Herausfiltern von Partikeln, Fasern oder dgl., die die nachgeordnete Austragsdüse 12 oder einen Feinfilter verstopfen oder verlegen könnten. Die Filterschwelle liegt vorzugsweise im Bereich von etwa 10 µm. Größere Partikel, Teilchen, Fasern oder dgl. werden also aus der durchströmenden Arzneimittelzubereitung 2 vom Vorfilter 27 herausgefiltert bzw. zurückgehalten.

Der Vorfilter 27 ist vorzugsweise aus einem gegenüber der Arzneimittelzubereitung 2 chemisch verträglichen Kunststoff, insbesondere einem Polyolefin, besonders bevorzugt Polyethylen oder Polypropylen, hergestellt. Beim Darstellungsbeispiel handelt es sich insbesondere um Polyethylen-Sintermaterial.

Beim Darstellungsbeispiel wird der Vorfilter 27 von einem Filterhalter 28 unmittelbar am auslaßseitigen Ende der Druckkammer 11 - vorzugsweise gepreßt - gehalten, wobei der Filterhalter 28 mittels einer Dichtung 29, insbesondere eines O-Rings, gegenüber dem Zentralteil 23 abgedichtet wird. Jedoch sind auch andere konstruktive Lösungen möglich.

An den Vorfilter 27 bzw. Filterhalter 28 schließt sich vorzugsweise umnittelbar ein Feinstfilter und/oder die Austragsdüse 12 an. Besonders bevorzugt erfolgt eine zweistufige Filterung der Arzneimittelformulierung 2 über Vorfilter 27 und Feinstfilter vor der Zerstäubung. Insbesondere bildet die Austragsdüse 12 den Feinstfilter, wie später noch näher erläutert.

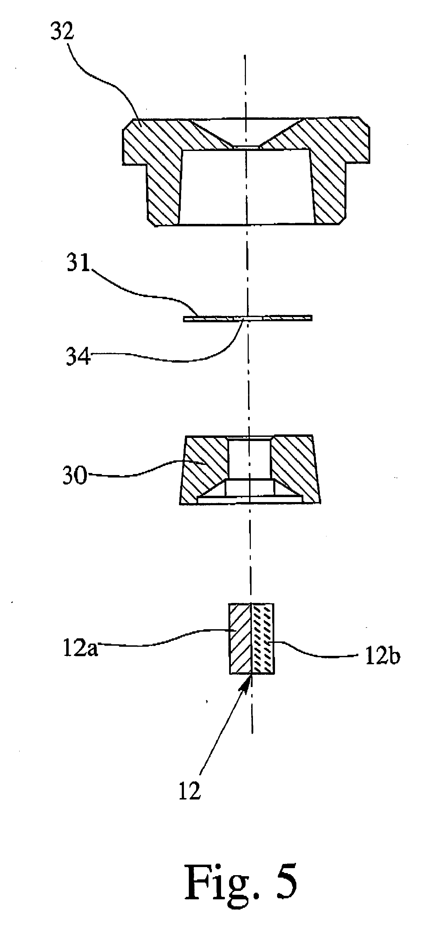

Die Austragsdüse 12 wird vorzugsweise von einer Düsendichtung 30 aufgenommen bzw. radial gehalten, wie aus der ausschnittsweisen, explosionsartigen Darstellung gemäß Fig. 5 ersichtlich.

Abgabeseitig schließen sich vorzugsweise ein Halteelement 31 sowie ein optionales Halteteil 32 an. Schließlich ist zur Befestigung am Zentralteil 23 ein weiteres Haltemittel, hier ein Düsenhalter bzw. eine Überwurfmutter 33, vorgesehen. Das Haltemittel dient insbesondere einer gemeinsamen Halterung des Vorfilters 27 und der Austragsdüse 12 und/oder einer gemeinsamen Halterung der Austragsdüse 12 und des nachgeordneten Halteelements 31, wie in den Fig. 1 bis Fig. 4 angedeutet. Jedoch sind auch andere konstruktive Lösungen möglich.

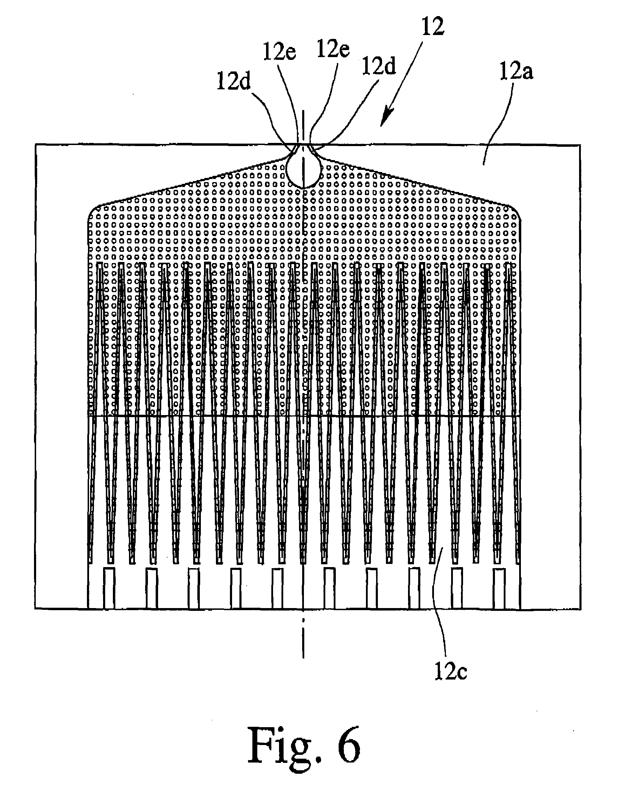

Die Austragsdüse 12 ist vorzugsweise aus zwei miteinander fest verbundenen Düsenteilen, insbesondere Platten 12a und 12b, aufgebaut bzw. hergestellt, die insbesondere aus Glas und/oder Silizium bestehen und von denen wenigstens eine mikrostrukiroert ist, wie beispielhaft in Fig. 6 dargestellt. Aufgrund dieser Mikrostrukturierung auf einer Flachseite wird ein Strömungsbereich 12c gebildet, der einen Düseneinlaß mit dem Düsenauslaß verbindet.

Die Austragsdüse 12 weist vorzugsweise zwei Auslaßkanäle 12d auf, die insbesondere unmittelbar vom Strömungsbereich 12c gebildet sind. Die Auslaßkanäle 12d bilden zusammen mit dem weiteren Düsenteil bzw. einer Abdekkung 12b Düsenöffnungen 12e der Austragsdüse 12, über die die zu zerstäubende Arzneimittelzubereitung 2 unter hohem Druck an die Umgebung ausgegeben wird.

Die Düsenöffnungen 12e weisen vorzugsweise eine Größe von etwa 2 bis 10 µm auf etwa 5 bis 15 µm, besonders bevorzugt von etwa 4,5 bis 6,5 µm auf etwa 7 bis 9 µm auf Jedoch sind auch andere Dimensionierungen möglich.

Grundsätzlich kann die Austragsdüse 12 auch lediglich nur einen Auslaßkanal 12d mit nur einer Düsenöffnung 12e aufweisen. Vorzugsweise sind jedoch mindestens zwei Auslaßkanäle 12d mit getrennten Düsenöffnungen 12e vorgesehen.

Die Auslaßkanäle 12d sind vorzugsweise zueinander geneigt. Insbesondere kreuzen sich die von den Auslaßkanälen 12d über die Düsenöffnungen 12e abgegebenen Strahlen der Arzneimittelzubereitung 2. Dies bewirkt oder unterstützt die Zerstäubung der Arzneimittelformulierung 2 in sehr kleine Tröpfchen bzw. Partikel.

Besonders bevorzugt schließen die Auslaßkanäle 12d bzw. die davon abgegebenen Strahlen einen Winkel von 20° bis 160°, insbesondere von 60° bis 150° und beim Darstellungsbeispiel von etwa 80° bis 100°, ein.

Die Düsenöffnungen 12e sind bevorzugt in einer Entfernung von 10 µm bis 200 µm, insbesondere etwa 10µm bis 100 µm, besonders bevorzugt etwa 30 µm bis 70 µm, angeordnet.

Die beiden Düsenteile bzw. Platten 12a, 12b sind vorzugsweise durch Bonden oder auf sonstige geeignete Art und Weise miteinander fest verbunden. Jedoch sind auch andere konstruktive Lösungen möglich.

Vorzugsweise ist das Volumen der sich an die Druckkammer 11 anschließenden fluidischen Verbindung zur Austragsdüse 12 und/oder das Volumen der Austragsdüse 12 selbst möglichst klein ausgebildet, um ein geringes Totvolumen zu erreichen.

Besonders bevorzugt ist der Strömungswiderstand durch die Austragsdüse 12, selbst für Luft, wesentlich höher, insbesondere mindestens um den Faktor 100, besonders bevorzugt um den Faktor 1000 oder mehr, als der Strömungswiderstand der durch das Förderrohr 9 beim Spannhub in die Druckkammer 11 nachströmenden Arzneimittelformulierung 2. Dies gestattet es, auf ein auslaßseitiges Ventil zu verzichten. Vielmehr wird beim Ansaugen (Spannhub) lediglich das Rückschlagventil 10 geöffnet. Die über die Austragsdüse 12 rückströmende Luft ist aufgrund der genannten Widerstandsverhältnisse vernachlässigbar.

Der genannte hohe Strömungswiderstand durch die Austragsdüse 12 wird insbesondere durch entsprechend kleindimensionierte Düsenöffnungen 12e erreicht.

Die feinen Düsenöffnungen 12e führen auch zu der gewünschten, sehr feinen Zerstäubung der Arzneimittelformulierung 2 beim Austrag.

Besonders bevorzugt weist die Austragsdüse 12 einen integrierten Feinstfilter auf. Dieser Feinstfilter ist insbesondere durch entsprechende Strukturierung des Strömungsbereichs 12c, beispielsweise feine Durchlässe durch eine Wandung und/oder Erhebungen mit bestimmter Flächendichte, gebildet, wie aus Fig. 6 ersichtlich. Jedoch kann der Feinstfilter auch durch ein separates Bauteil gebildet werden.

Mittels des Feinstfilters können Partikel herausgefiltert werden, die die sehr feinen Auslaßkanäle 12d verstopfen oder verlegen könnten. Die Filterschwelle liegt insbesondere bei 2 µm bis 5 µm.

Die Austragsdüse 12 ist vorzugsweise wie in der WO 94/07607 A1 und/oder WO 99/16530 A1 beschrieben ausgebildet.

Das Halteelement 31 ist insbesondere scheibenartig und/oder folienartig ausgebildet. Besonders bevorzugt handelt es sich um eine Art Zwischenlagscheibe mit einem Durchgangsloch 34, durch das die zerstäubte Arzneimittelzubereitung 2 hindurchtreten kann.

Das Halteelement 31 ist vorzugsweise aus einem weicheren Material, insbesondere Kunststoff, als die Austragsdüse 12 hergestellt. Das Halteelement 31 dient insbesondere einer sicheren Halterung der Austragsdüse 12 und wird durch das nachgeordnete Halteteil 32 bzw. die Überwurfmutter 33 auf die Austragsdüse 12 im montierten Zustand gepreßt. Weiter kann das Halteelement 31 auch einen gewissen Toleranzausgleich dienen.

Hinsichtlich des Förderrohrs 9 ist noch anzumerken, daß das Rückschlagventil 10 vorzugsweise einen Ventilkörper 35 umfaßt, wie aus Fig. 4 ersichtlich, der in einer entsprechenden endseitigen Ausnehmung des Förderrohrs 9 axial begrenzt beweglich gehalten ist.

Insbesondere ist der Ventilkörper 35 seitlich und/oder stirnseitig (zur Druckkammer 11 hin) mit einer Ausnehmung, Nut oder dergleichen versehen, so daß der Ventilkörper 35 bei geöffnetem Rückschlagventil 10 von der Arzneimittelzubereitung 2 umstömbar ist, auch wenn der Ventilkörper 35 abströmseitig an einem axialen, insbesondere ringschulterartigen Anschlag des Förderrohrs 9 bei geöffnetem Rückschlagventil 10 zur Anlage kommt. Jedoch sind auch andere konstruktive Lösungen möglich.

Es ist anzumerken, daß das Förderrohr 9 insbesondere als dickwandige Kapillare ausgebildet ist. Insbesondere kann so ein geringes Totvolumen im Förderrohr 9 erreicht werden.

Das Förderrohr 9 ist fest mit der Halterung 6 verbunden. Insbesondere ist die Halterung 6 hierzu direkt an das Förderrohr 9 angespritzt. Jedoch sind auch andere konstruktive Lösungen möglich.

Die Antriebsfeder 7 ist vorzugsweise als zylindrische Schraubenfeder ausgebildet. Sie ist insbesondere unmittelbar mit einem Ende an der Halterung 6 abgestützt. Die Halterung 6 weist hierzu beispielsweise eine Ringschulter 36 auf.

Das andere Ende der Antriebsfeder 7 ist vorzugsweise über einen Verschlußring 37 im Bereich des unteren Endes am Innenteil 17, insbesondere am unteren Teil 17b des Innenteils 17, abgestützt. Hierzu ist der Verschlußring 37 vorzugsweise selbstsichernd, insbesondere rastend oder einklippbar, am Innenteil 17 anbringbar.

Die Antriebsfeder 7 ist vorzugsweise vorgespannt eingebaut, um einen hohen Förderdruck zu erreichen. Beim vorschlagsgemäßen Zerstäuber 1 erfolgt das Unterdrucksetzen und Fördern der Arzneimittelzubereitung 2 während des Zerstäubungsvorgangs nämlich vorzugsweise nur durch Federkraft, insbesondere nur durch die Kraft der Antriebsfeder 7.

Der Zerstäuber 1 ist vorzugsweise derart ausgebildet, daß die Arzneimittelzubereitung 2 im Druckerzeuger 5 bzw. in der Druckkammer 11 bei der Ausgabe einem Druck von 5 MPa bis 60 MPa, insbesondere etwa 10 MPa bis 50 MPa, durch die Kraft der Antriebsfeder 7 erreicht. Besonders bevorzugt wird bei der Ausgabe bzw. Zerstäubung der Arzneimittelzubereitung 2 ein Druck von etwa 5 MPa bis 60 MPa, insbesondere etwa 10 bis 30 MPa, an der Austragsdüse 12 bzw. an deren Düsenöffnungen 12e erreicht. Die Arzneimittelzubereitung 2 wird dann in das Aerosol 14 überfuhrt, dessen Tröpfchen einen aerodynamischen Durchmesser von bis zu 20 µm, bevorzugt etwa 3 µm bis 10 µm, aufweisen. Die Zerstäubungswirkung bzw. der Zerstäubungseffekt wird durch die sich vorzugsweise kreuzenden Strahlen, die von der Austragsdüse 12 abgegeben werden, bewirkt bzw. weiter unterstützt.

Die Menge der pro Hub ausgebrachten Arzneimittelzubereitung 2 beträgt vorzugsweise etwa 10 µl bis 50 µl, insbesondere etwa 10 µl bis 20 µl, ganz besonders bevorzugt etwa 15 µl.

Die Halterung 6 ist axial verschieblich im Innenteil 17 geführt, aber drehfest mit dem Innenteil 17 gekoppelt. Dies wird beispielsweise durch eine entsprechende axiale Nut in der Halterung 6 und/oder im Innenteil 17 und einen radialen Eingriff erreicht.

Bei Drehen des zweiten Gehäuseteils 18 wird das Innenteil 17 mitgedreht. Hierdurch wird der Zerstäuber 1 bzw. dessen Antriebsfeder 7 gespannt. Dies erfolgt über ein entsprechendes Getriebe, insbesondere ein Schraub-Schub-Getriebe oder Keilgetriebe, beim Darstellungsbeispiel über entsprechend geneigte, insbesondere schraubenlinienförmige Nocken- bzw. Abgleitfläche 38, die an der Halterung 6 und/oder am ersten Gehäuseteil 16 gebildet und nur schematisch in Fig. 2 bis Fig. 4 angedeutet sind. Wenn die Halterung 6 relativ zum ersten Gehäuseteil 16 gedreht wird (in die Spanndrehriohtung, ein Drehen in die entgegengesetzte Richtung ist vorzugsweise gesperrt), wird die Halterung 6 zusammen mit dem Förderrohr 9 und dem Behälter 3 axial zum zweiten Gehäuseteil 18 - bei den Darstellungen nach unten - gegen die Kraft der Antriebsfeder 7 verschoben, wodurch die Antriebsfeder 7 entsprechend gespannt wird.

Es ist anzumerken, daß zum Spannen des Zerstäubers 1 das zweite Gehäuseteil 18 relativ zum ersten Gehäuseteil 16 jeweils um einen bestimmten Drehwinkel drehbar ist, bis der Zerstäuber 1 gespannt ist und das Sperrelement 8 vorzugsweise selbsttätig einrastet. Dieser Drehwinkel ist vorzugsweise ein ganzteiliger Bruchteil von 360° und beträgt beispielsweise 180°,

Die Halterung 6 führt bei der genannten Spannbewegung einen gewissen Überhub aus, wobei das Sperrelement 8 bei Erreichen der Überhubposition selbsttätig eine insbesondere radial verschobene Sperrposition einnimmt und beim Erreichen einer Enddrehposition die Halterung 6 nicht mehr von dem Getriebe bzw. den Nocken- oder Abgleitflächen 38, sondern nur noch von dem Sperrelement 8 in axialer Richtung gegen ein Zurückbewegen gehalten wird.

Das Sperrelement 8 weist vorzugsweise einen Sperring 39 auf, durch den sich die Halterung 6 im nicht gesperrten Zustand axial hindurch nach oben in Richtung des Mundstücks 13 bewegen kann. In der Sperrposition ist der Sperring 39 nicht koaxial angeordnet, sondern radial verschoben, so daß die Halterung 6 insbesondere stirnseitig am Sperring 39 axial anliegt und dementsprechend der Zerstäubungs- bzw. Förderhub blockiert ist.

Das Sperrelement 8 bzw. der Sperring 39 ist vorzugsweise mit einer zugeordneten Taste 40 versehen. Die Taste 40 ist insbesondere fest mit dem Sperring 39 verbunden, beispielsweise auf diesem aufgesteckt. In der Sperrposition ist die Taste 40 vorzugsweise etwas radial ausgerückt, wie in Fig. 3 angedeutet. In Fig. 2 ist das Sperrelement 8 bzw. der Sperring 39 noch nicht radial in die Sperrposition verschoben.

Durch manuelles Eindrücken der Taste 40 ist das Sperrelement 8 bzw. der Sperring 39 wieder radial verschiebbar, so daß die Halterung 6 in axialer Richtung freigegeben wird, wodurch der Zerstäuber 1 betätigt bzw. die Zerstäubung der Arzneimittelformulierung 2 ausgelöst oder freigegeben wird. Bei dem damit ausgelösten Zerstäubungs- bzw. Förderhub wird dann das Förderrohr 9 durch die Kraft der Antriebsfeder 7 in die Druckkammer 11 hineingedrückt und dementsprechend die darin befindliche Arzneimittelzubereitung 2 über die Austragsdüse 12 unter hohem Druck ausgetragen und zerstäubt.

Hinsichtlich des bevorzugten Aufbaus und der Funktion des Sperrelements 8 wird ergänzend auf die WO 97/20590 A1 verwiesen.

Der Zerstäuber 1 weist vorzugsweise eine Zähleinrichtung 41 zur Zählung von Betätigungen des Zerstäubers 1 auf. Insbesondere ist die Zähleinrichtung 41 derart ausgebildet, daß der Zerstäuber 1 gegen ein erneutes Betätigen gesperrt wird, wenn eine bestimmte Anzahl von Betätigungen des Zerstäubers 1 erreicht oder überschritten wird (Endsperrzustand). Insbesondere wird hier das Spannen des Zerstäubers bzw. der Antriebsfeder 7 als Betätigen verstanden.

Die Zähleinrichtung 41 ist beim Darstellungsbeispiel durch die Relativdrehung des Innenteils 17 zum ersten Gehäuseteil 16 angetrieben. Entsprechend wird diese Relativdrehung als Betätigung des Zerstäubers 1 erfaßt und gezählt. Jedoch sind auch andere konstruktive Lösungen möglich.

Die Zähleinrichtung 41 weist beim Darstellungsbeispiel eine Gewindespindel 42 mit einem zugeordneten, durch Drehen der Gewindespindel 42 insbesondere linear bewegbaren Reiter 43 auf, wie in Fig. 1 und Fig. 4 angedeutet.

Die Zähleinrichtung 41 ist vorzugsweise am drehbaren Innenteil 17 des Zerstäubers 1 angeordnet.

Die Gewindespindel 42 ist vorzugsweise im wesentlichen parallel zur Drehachse des Innenteils 17 und vorzugsweise außen am Mantel des Innenteils 17 im Bereich des unteren Teils 17b angeordnet und drehbar gelagert. Es ist anzumerken, daß die Gewindespindel 42 vorzugsweise in eine entsprechende Führung bzw. Drehlager am Innenteil 17 seitlich einrastbar bzw. einklippbar ist.

Der Reiter 43 ist außen am Innenteil 17 längs verschiebbar von der Gewindespindel 42 geführt.

Insbesondere bildet der Reiter 43 mit der Gewindespindel 42 ein selbsthemmendes Schneckengetriebe.

Die Gewindespindel 42 wird vorzugsweise durch innen am ersten Gehäuseteil 16 angebrachte bzw. gebildete Zähne 16a angetrieben bzw. gedreht. Insbesondere kämmen die in Fig. 4 angedeuteten Zähne 16a mit einem sich in das erste Gehäuseteil 16 erstreckenden Abschnitt 42a der Gewindespindel 42, der auch eine entsprechende Verzahnung bzw, Eingriffsmöglichkeit für die Zähne 16a aufweist.

Beim Darstellungsbeispiel weist das erste Gehäuseteil 16, vorzugsweise vier Zähne 16a über den Umfang verteilt auf. Besonders bevorzugt sind die Zähne 16a derart über den Umfang des ersten Gehäuseteils 16 verteilt, daß die Zähleinrichtung 41 bei jedem Spannhub - also bei jedem Verdrehen des Innenteils 17 zum ersten Gehäuseteil 16 - angetrieben wird, die Gewindespindel 42 also jeweils weitergedreht wird.

Insbesondere erfolgt das Drehen des Innenteils 17 relativ zum ersten Gehäuseteil 16 in Schritten von 180° für jeden Spannhub. Die Zähne 16a sind dann derart verteilt, insbesondere im wesentlichen diametral einander gegenüberliegend am ersten Gehäuseteil 16 angeordnet, daß bei jedem Drehschritt des Innenteils 17 die Gewindespindel 42 auch weitergedreht wird.

Besonders bevorzugt ist der Abschnitt 42a der Gewindespindel 42, der mit den Zähnen 16a kämmt, derart ausgebildet und auf die Anzahl der Zähne 16a abgestimmt, daß die Gewindespindel 42 bei jedem Drehschritt (180°-Drehung des Innenteils 17 relativ zum ersten Gehäuseteil 16) um eine halbe Umdrehung weitergedreht wird. Besonders bevorzugt weist der Zerstäuber 1 insgesamt vier am Gehäuseteil 16 angeordnete Zähne 16 auf. Diese sind vorzugsweise in zwei Paaren diametral gegenüberliegend und/oder jeweils kurz vor dem Erreichen des Endes des jeweiligen Drehschritts am ersten Gehäuseteil 16 angeordnet. Der Abschnitt 42a weist dann entsprechend vier über seinen Umfang verteilte Eingriffsmöglichkeiten oder Zähne auf, die mit den Zähnen 16a kämmen.

Insbesondere ist die Zähleinrichtung 41 beim Darstellungsbeispiel derart ausgebildet, daß etwa 60 bis 70 einzelne Betätigungen des Zerstäubers 1 möglich sind. Diese Anzahl schließt beispielsweise einige Betätigungen zum Primen und das anschließende Ausbringen der Arzneimittelzubereitung 2 zur Inhalation ein.

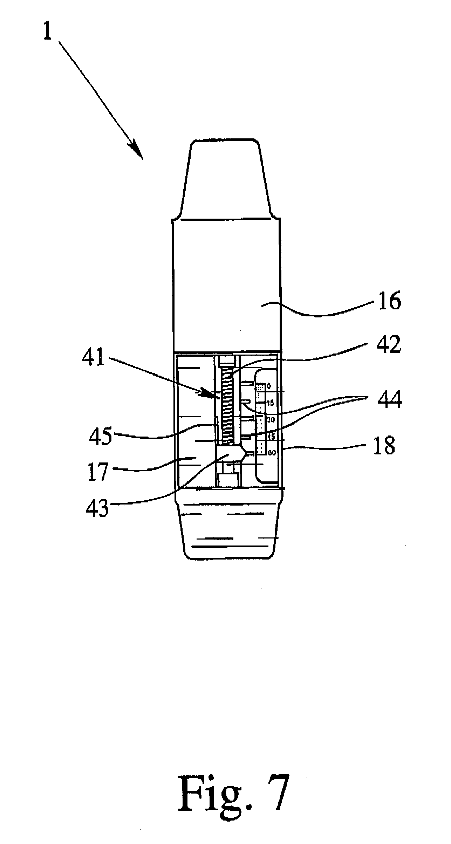

Fig. 7 zeigt in einer schematischen Seitenansicht den Zerstäuber 1. Das zweite Gehäuseteil 18 ist vorzugsweise zumindest partiell durchsichtig bzw, transparent ausgebildet, so daß zumindest wesentliche Teile des Zählwerks 41, wie der Reiter 43, erkennbar sind.

Beim Darstellungsbeispiel ist das zweite Gehäuseteil 18 vorzugsweise insgesamt durchsichtig bzw. transparent ausgebildet, insbesondere aus einem transparenten Kunststoff hergestellt. Dies gestattet auch unabhängig von der Sichtbarkeit der Zähleinrichtung 41 die Abringung von Informationen auf dem unteren Teil 17b des Innenteils 17, die von außen sichtbar sind. Insbesondere können die Informationen durch einen Aufkleber oder mehrere Aufkleber auf dem Innenteil 17 angebracht werden. Diese sind dann durch das zweite Gehäuseteil 18 geschützt.

Fig. 7 ist zu entnehmen, daß die Zähleinrichtung 41 vorzugsweise dem Reiter 43 zugeordnete Zählmarkierungen 44 aufweist. Diese sind beim Darstellungsbeispiel insbesondere durch Nuten am Innenteil 17 gebildet. Jedoch können diese alternativ oder zusätzlich auch durch entsprechende neben dem Reiter 43 angebrachte, insbesondere aufgeklebte Markierungen oder dgl. gebildet sein.

Vorzugsweise geben die Zählmarkierungen 44 die noch möglichen Betätigungen des Zerstäubers 1 - genauer gesagt, die Anzahl der noch auszugebenden Dosen - bis zum Erreichen des Endsperrzustands an.

Der Reiter 43 ist beim Darstellungsbeispiel vorzugsweise mit einem Betätigungsarm 45 versehen, der sich insbesondere parallel zur Gewindespindel 42 erstreckt und über die Gewindespindel 42 axial hinausreicht, wenn der Reiter 43 seine in der Darstellung gemäß Fig. 7 obere Endstellung entlang der Gewindespindel 42 erreicht, Der Betätigungsarm 45 dient nämlich insbesondere einer Betätigung einer Blockiereinrichtung im Endsperrzustand.

Die Blockiereinrichtung wird beim Darstellungsbeispiel durch eine Sperrfeder 46 gebildet, die in Fig. 1 und Fig. 4 angedeutet ist. Die Sperrfeder 46 ist insbesondere als gefaltete und/oder zweischenklige Blattfeder ausgebildet. Die Sperrfeder 46 ist vom Innenteil 17 gehalten, und zwar vorzugsweise in einer nach außen offenen Längsnut. Ein Schenkel der Sperrfeder 46 liegt vorzugsweise am Nutboden an. Der andere Schenkel ist in radialer Richtung vorgespannt und hinter einem Vorsprung am Innenteil 17 oder von einem Ringbereich des ersten Gehäuseteils 16 gegen ein radiales Ausfedern nach außen gehalten.

Wenn die vorbestimmte bzw. maximale Anzahl von Betätigungen des Zerstäubers 1 erreicht oder überschritten wird und der Reiter 43 seine Endstellung einnimmt, verschiebt der Reiter 43 mit seinem Betätigungsarm 45 die Sperrfeder 46 axial (in Fig. 1 nach oben), so daß der vorgespannte Schenkel freigegeben wird und radial nach außen in eine entsprechende Nut bzw, Ausnehmung 47 innen am ersten Gehäuseteil 16 ausfedern oder eingreifen kann. Alternativ kann der ausfedernde Schenkel der Sperrfeder 46 auch als Widerlager oder Anlage für einen entsprechenden Drehanschlag oder Vorsprung des ersten Gehäuseteils 16 dienen, um eine weitere Relativdrehung zwischen dem Innenteil 17 und dem ersten Gehäuseteil 16 zu blockieren. Damit wird der Endsperrzustand erreicht. Ein weiteres Drehen des Innenteils 17 relativ zum ersten Gehäuseteil 16 wird also endgültig - zumindest in Spannrichtung - blockiert. Damit wird eine weitere Betätigung des Zerstäubers 1 bzw. ein weiteres Spannen des Zerstäubers 1 blockiert.

Hinsichtlich einer bevorzugten Ausgestaltung der Blockierung des erneuten Spannens bzw. Drehens des Innenteils 17 bzw. zweiten Gehäuseteils 18 relativ zum ersten Gehäuseteil 16 wird ergänzend auf die WO 2004/024340 A1 verwiesen.

Der vorschlagsgemäße Zerstäuber 1 ist insbesondere derart ausgebildet, daß er zur Ausbringung einer Dosis der Arzneimittelzubereitung 2 zweimal hintereinander betätigt, nämlich zweimal hintereinander gespannt und ausgelöst, werden muß, Das Spannen und Auslösen erfolgt abwechselnd. Diese zweimalige Betätigung wird nur als eine Betätigung oder nur als eine vom Zerstäuber 1 abgegebene Dosis von der Zähleinrichtung 41 erfaßt oder gezählt. Jede Dosis der Arzneimittelzubereitung 2 wird also durch einen Doppelhub der Fördereinrichtung der Pump- bzw. Fördereinrichtung des Zerstäubers 1 bzw. des Druckerzeugers 5 ausgegeben. So ist es insbesondere möglich, auch größere Dosismengen unter den gewünschten Zerstäubungsbedingungen und - parametern auszugeben.

Der Zerstäuber 1 ist vorzugsweise derart ausgebildet, daß insgesamt 30 bis 35 Dosen, insbesondere 33 oder 34 Dosen, der Arzneimittelzubereitung 2 ausgebbar sind. Dementsprechend ist der Zerstäuber 1 vorzugsweise derart ausgebildet, daß insgesamt 60 bis 70, insbesondere 66 bis 68, Förder- und Spannhübe bzw. Auslösungen, also einzelne Zerstäubungen, durchführbar sind (da jeweils ein Doppelhub bzw. ein zweimaliges Spannen und Auslösen zur Ausgabe und Zerstäubung einer Dosis der Arzneimittelzubereitung 2 erforderlich ist), bevor der Endsperrzustand durch Blockierung durch die Sperrfeder 46 erreicht wird.

Besonders bevorzugt weist der Zerstäuber 1 bzw. Behälter 3 eine derartige Menge der Arzneimittelzubereitung 2 auf, daß bei den vorzugsweise vorgesehenen 60 bis 70 Einzelbestätigungen dann entsprechend 30 bis 35 Dosen der Arzneimittelzubereitung 2 (zwei Bestätigungen bzw. Förderhübe bilden eine Dosis) ausgebbar sind.

Der Zerstäuber 1 weist vorzugsweise eine Mundstückabdeckung 48 auf. Diese ist nur in Fig. 3 und Fig. 4 dargestellt. Die Mundstückabdeckung 48 ist insbesondere als schwenkbare Kappe oder dgl. ausgebildet.

Zur Vervollständigung der Offenbarung der vorliegenden Patentanmeldung und zur bevorzugten Ausbildung des Zerstäubers 1 wird vorsorglich auf den kompletten Offenbarungsgehalt sowohl der WO 91/14468 A1 als auch der WO 97/12687 A1 verwiesen.

Im Gegensatz zu Standgeräten oder dergleichen ist der vorschlagsgemäße Zerstäuber 1 vorzugsweise transportabel ausgebildet, insbesondere handelt es sich um ein mobiles Handgerät.

Aufgrund seiner zylinderähnlichen Form und einer handlichen Größe von weniger als 9 bis 15 cm in der Länge und 2 bis 4 cm in der Breite kann der Zerstäuber 1 jederzeit vom Patienten mitgeführt werden. Der Vernebler versprüht ein definiertes Volumen der Arzneimittelzubereitung 2 unter Anwendung hoher Drücke durch kleine Düsen, so daß inhalierbare Aerosole 14 entstehen.

Der vorschlagsgemäße Zerstäuber 1 arbeitet insbesondere rein mechanisch. Jedoch kann der Zerstäuber 1 grundsätzlich auch auf jede sonstige Art und Weise arbeiten. Insbesondere ist der Begriff "Fördereinrichtung" bzw. "Druckerzeuger" sehr allgemein zu verstehen. Beispielsweise kann der zur Ausgabe und Zerstäubung erforderliche Druck auch durch Treibgas, eine Pumpe oder auf jede sonstige geeignete Art und Weise erzeugt werden.

Der vorschlagsgemäße Zerstäuber 1 ist insbesondere zur kurzzeitigen Zerstäubung der Arzneimittelzubereitung 2, beispielsweise für ein bis zwei Atemzüge, ausgebildet. Jedoch kann er auch zur längeren oder kontinuierlichen Zerstäubung ausgebildet bzw. einsetzbar sein.

Einzelne Aspekte und Merkmale des Zerstäubers 1 bzw. der beschriebenen Varianten können auch beliebig miteinander kombiniert werden.

Nachfolgend werden bevorzugte Verbindungen, Bestandteile und/oder Formulierungen der Arzneimittelzubereitung 2 aufgeführt.

Der Zerstäuber 1 enthält eine treibgasfreie Aerosolformulierung von einem in Wasser gelöstem pharmazeutisch akzeptablem Salz von Tiotropium. Die erfindungsgemäße Formulierung eignet sich besonders zum Vernebeln des Wirkstoffs mittels des Zerstäubers 1, um den Wirkstoff inhalativ zu applizieren. Bevorzugte Indikationen sind Asthma und/oder COPD.

Tiotropium, chemisch (1α,2β,4β,5α,7β)-7-[(hydroxydi-2-thienylacetyl)oxy]-9,9-dimethyl-3-oxa-9-azoniatricyclo[3.3.1.0]nonane, ist als Tiotropiumbromid aus der Europäischen Patentanmeldung EP 418 716 A1oder US 5,610,163 bekannt. Das Bromid-Salz des Tiotropiums weist die folgende chemische Struktur auf: 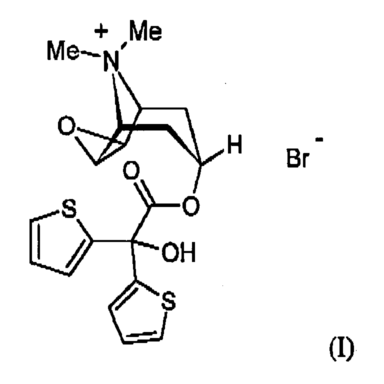 Die Verbindung besitzt wertvolle pharmakologische Eigenschaften und ist unter dem Namen Tiotropiumbromid bekannt. Tiotropium und seine Salze stellen hochwirksame Anticholinergika dar und können deshalb bei der Therapie von Asthma oder COPD (chronic obstructive pulmonary disease = chronisch obstruktive Lungenerkrankung) einen therapeutischen Nutzen entfalten.

Diese Verbindungen sind bevorzugter Gegenstand der vorliegenden Erfindung.

Der Zerstäuber 1 bzw. die Arzneimittelzubereitung 2 inhalativ applizierbare flüssige Wirkstoffformulierungen dieser Verbindungen, wobei die flüssigen Formulierungen hohen Qualitätsstandards genügen müssen.

Um eine optimale Wirkstoffverteilung der Wirksubstanzen in der Lunge zu erhalten, bietet sich die Applikation einer flüssigen, auf Treibgase verzichtenden Formulierung mittels des Zerstäubers 1 an. Besonders geeignet wird insbesondere eine kleine Menge einer flüssigen Formulierung in der therapeutisch notwendigen Dosierung binnen weniger Sekunden in ein therapeutischinhalativ geeignetes Aerosol vernebelt. Im Rahmen der vorliegenden Erfindung wird vorzugsweise eine Menge von weniger als 100 Mikroliter, bevorzugt weniger als 50 Mikroliter, ganz bevorzugt weniger als 20 Mikroliter, Wirkstofflösung mit bevorzugt einem Hub oder zwei Hüben zu einem Aerosol mit einer durchschnittlichen Teilchengröße von weniger als 20 Mikrometern, bevorzugt weniger als 10 Mikrometern, so vernebelt, daß der inhalierbare Anteil des Aerosols bereits der therapeutisch wirksamen Menge entspricht.

Die Lösungsformulierungen werden in einem Reservoir gelagert. Dabei ist es notwendig, daß die verwendeten Wirkstoffformulierungen eine ausreichende Lagerstabilität aufweisen und gleichzeitig so beschaffen sind, daß sie dem medizinischen Zweck entsprechend möglichst ohne weitere Manipulation, direkt appliziert werden können. Ferner dürfen sie keine Bestandteile aufweisen, die so mit dem Zerstäuber 1 (Inhalator) wechselwirken können, daß der Inhalator oder die pharmazeutische Qualität der Lösung, respektive des erzeugten Aerosols, Schaden nehmen könnte.

Die WO 98/27959 offenbart Lösungsformulierungen für den oben beschriebenen Inhalator, die als Zusatz das Dinatriumsalz der Editinsäure (bzw. Dinatrium- ethylendiamintetraacetat-Dihydrat oder Natriumedetat) enthalten. Die Schrift favorisiert für wässrige Lösungsformulierungen, die mit Hilfe des eingangs beschriebenen Inhalators in inhalierbare Aerosole versprüht werden sollen, eine Mindestkonzentration an Natriumedetat von 50 mg/100 ml, um die Inzidenz von Sprühanomalien zu verringern. Unter den offenbarten Beispielen findet sich eine Formulierung mit Tiotropiumbromid mit einem pH-Wert von 3,2 bzw, 3,4. Bei dieser Formulierung ist der Wirkstoff in Wasser gelöst. Der Anteil an Natriumedetat beträgt ebenfalls 50 mg / 100 ml.

Überraschend wurde jetzt allerdings gefunden, daß wässrige Lösungsformulierungen von Tiotropiumsalzen dann besonders stabil sind, wenn der pH-Wert unter 3,2, bevorzugt unter 3,1 liegt.

Darüber hinaus wurde gefunden, dass derartige Formulierungen gegenüber der aus dem Stand der Technik bekannten Formulierung mit Tiotropiumbromid bei Vernebelung mittels des beschriebenen Inhalators eine Reduktion der Streuung der ausgebrachten Masse aufweist, wenn die Menge an Natriumedetat von 5 mg bis 20 mg pro 100 g Formulierung liegt. Die Sprayqualität der erfindungsgemäßen Formulierung ist sehr gut. Ein so generiertes Aerosol weist für die inhalative Applikation sehr gute Eigenschaften auf.

Außerdem weist die erfindungsgemäße Formulierung eine verbesserte Stabilität auf und vermindert die Belastung des Patienten mit Natriumedetat.

Die vorliegende Erfindung sieht eine wässrige Wirkstoffformulierung mit einem pharmazeutisch akzeptablen Tiotropiumsalz vor, welche den hohen Standards genügt, die notwendig sind, um eine Lösung mittels des eingangs genannten Inhalators optimal vernebeln zu können. Die erfindungsgemäßen Wirkstoffformulierungen müssen dabei auch eine ausreichende pharmazeutische Qualität aufweisen, d.h. sie sollten über eine Lagerzeit von einigen Jahren, bevorzugt von mindestens einem Jahr, stärker bevorzugt von zwei Jahren pharmazeutisch stabil sein.

Ein weiterer Aspekt besteht darin, treibgasfreie Lösungsformulierungen mit Tiotropiumsalzen zu schaffen, die mittels des Inhalators unter Druck vernebelt werden, wobei die im generierten Aerosol ausgebrachte Masse reproduzierbar innerhalb eines definierten Bereichs liegt.

Eine weitere Aufgabe besteht darin, eine inhalierbare Formulierung mit einem Tiotropiumsalz als flüssige Formulierung mit Wasser als Lösungsmittel zu schaffen, welche stabil ist und die Belastung des Patienten mit chemischen Stoffen auf ein Minimum reduziert.

Erfindungsgemäß können für die Formulierung alle pharmazeutisch akzeptablen Salze des Tiotropiums eingesetzt werden. Wird im Rahmen der vorliegenden Erfindung der Begriff Tiotropiumsalz verwendet, so ist dies als Bezugnahme auf Tiotropium zu verstehen. Eine Bezugnahme auf Tiotropium entspricht dem freien Ammoniumkation. Das Tiotropiumsalz enthält entsprechend ein Anion als Gegenion. Als im Rahmen der vorliegenden Erfindung einsetzbare Tiotropiumsalze sind bevorzugt Verbindungen zu verstehen, die neben Tiotropium als Gegenion (Anion) Chlorid, Bromid, Iodid, Methansulfonat, para-Toluolsulfonat und/oder Monomethylsulfat enthalten.

Zur Herstellung der erfindungsgemäßen Formulierungen ist als Salz das Tiotropiumbromid bevorzugt. Bezugnahmen auf Tiotropiumbromid sind im Rahmen der vorliegenden Erfindung stets als Bezugnahmen auf alle möglichen amorphen und kristallinen Modifikationen des Tiotropiumbromids zu verstehen. Diese können beispielsweise in der kristallinen Struktur Lösemittelmoleküle mit einschließen. Von allen kristallinen Modifikationen des Tiotropiumbromids sind erfindungsgemäß diejenigen, die Wasser mit einschließen (Hydrate) bevorzugt. Besonders bevorzugt ist im #rahmen der vorliegenden Erfindung das Tiotropiumbromid-Monohydrat einsetzbar.

Die Formulierung enthält bevorzugt keinen weiteren Wirkstoff, der nicht Tiotropium enthält oder ein pharmazeutisch verträgliches Salz davon ist.

In der erfindungsgemäßen Formulierung (Arzneimittelzubereitung 2) liegt (liegen) das Tiotropiumsalz (die Tiotropiumsalze) in Wasser gelöst vor. Ein weiteres Lösungsmittel wird nicht verwendet. Die Formulierung ist insbesondere frei von Treibgasen.

Erfindungsgemäß enthält die Formulierung (Arzneimittelzubereitung 2) bevorzugt nur ein einziges Tiotropiumsalz, bevorzugt Tiotropiumbromid oder Tiotropiumbromid-Monohydrat. Allerdings kann die Formulierung auch ein Gemisch verschiedener Tiotropiumsalze und Solvate enthalten.

Die Konzentration des Tiotropiumsalzes bezogen auf den Anteil an Tiotropium in der fertigen Arzneimittelzubereitung ist abhängig von dem angestrebten therapeutischen Effekt. Für die Mehrzahl der auf Tiotropium ansprechenden Erkrankungen liegt die Konzentration an Tiotropium zwischen 0,01 g pro 100 g Formulierung und 0,06 g pro 100 g Formulierung. Da die Dichte der Formulierung 1,00 g/cm³ liegt, entsprechen die 100 g Formulierung einem Volumen von 100 ml. Im Rahmen der vorliegenden Erfindungsbeschreibung bedeutet die Bezeichnung pro 100 ml bzw. /100 ml jeweils pro 100 ml Formulierung, sofern nicht anders gekennzeichnet. Bevorzugt ist eine Menge von 0,015 g /100 ml bis 0,055 g / 100 ml, stärker bevorzugt ist eine Menge von 0,02 g / 100 ml bis 0,05 g / 100 ml. Am stärksten bevorzugt ist eine Menge von 0,023 + 0,001g pro 100 ml Formulierung bis zu 0,045 + 0,001g pro 100 ml Formulierung.

Der pH-Wert der erfindungsgemäßen Formulierung liegt zwischen 2,7 und 3,1, bevorzugt zwischen 2,8 und 3,05, stärker bevorzugt zwischen 2,80 und 3,0 und noch stärker bevorzugt bei 2,9.

Der pH-Wert wird durch Zugabe von pharmakologisch verträglichen Säuren eingestellt. Beispiele für diesbezüglich bevorzugte anorganische Säuren sind: Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit dem Wirkstoff ein Säureadditionssalz bilden.

Unter den organischen Säuren sind Ascorbinsäure, Fumarsäure und Zitronensäure bevorzugt, wobei Zitronensäure am stärksten bevorzugt ist. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe oder Antioxidantien besitzen, wie beispielsweise Zitronensäure oder Ascorbinsäure.

Unter den genannten Säuren werden ausdrücklich Salzsäure und Zitronensäure als am Stärksten bevorzugt genannt.

Gegebenenfalls können auch pharmakologisch verträgliche Basen zum genauen Austitrieren des pH-Wertes eingesetzt werden. Als Basen eignen sich beispielsweise Alkalihydroxide und Alkalicarbonate. Bevorzugtes Alkaliion ist Natrium. Werden solche Basen verwendet, ist darauf zu achten, daß auch die daraus resultierenden Salze, die dann in der fertigen Arzneimittelzubereitung enthalten sind, mit der oben genannten Säure pharmakologisch akzeptabel sind.

Erfindungsgemäß enthält die Formulierung Editinsäure (EDTA) oder ein bekanntes Salze davon, z.B. Natrium-EDTA, bzw. Dinatrium-EDTA-Dihydrat (Natriumedetat) als Stabilisator oder Komplexbildner. Bevorzugt wird Natriumedetat eingesetzt.

Der Gehalt bezogen auf Natriumedetat liegt dabei zwischen 5 mg / 100 ml Formulierung und 20 mg / 100 ml Formulierung, bevorzugt zwischen 5 mg / 100 ml Formulierung und 15 mg / 100 ml Formulierung, stärker bevorzugt zwischen 8 mg /100 ml Formulierung und 12 mg / 100 ml Formulierung, am stärksten bevorzugt bei 10 mg / 100 ml Formulierung.

Wird ein anders Salz der Editinsäure verwendet oder die Säure als solche, werden analoge Mengen des Komplexbildner eingesetzt.

Analoges wie bereits für Natriumedetat ausgeführt, gilt auch für möglich, wenn auch gegenüber EDTA oder seinen Salzen nicht bevorzugte andere vergleichbare Zusatzstoffe, die komplexbildende Eigenschaften aufweisen und anstelle dessen verwendet werden können, wie beispielsweise Nitrilotriessigsäure und deren Salze.

Unter Komplexbildner werden im Rahmen der vorliegenden Erfindung bevorzugt Moleküle verstanden, die in der Lage sind Komplexbindungen einzugehen. Bevorzugt sollen durch diese Verbindungen Kationen, besonders bevorzugt metallische Kautionen komplexiert werden.

Der erfindungsgemäßen Formulierung können weitere pharmakologisch verträgliche Hilfsstoffe zugesetzt werden.

Unter Hilfs- und Zusatzstoffen wird in diesem Zusammenhang jeder pharmakologisch verträgliche und therapeutisch sinnvolle Stoff verstanden, der kein Wirkstoff ist, aber zusammen mit dem Wirkstoff in dem pharmakologisch geeigneten Lösungsmittel formuliert werden kann, um die qualitativen Eigenschaften der Wirkstofffermulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. weitere Stabilisatoren, Komplexbildner, Antioxidantien und/oder Konservierungsstoffe, die die Verwendungsdauer der fertigen Arzneimittelzubereitung verlängern, Cresohmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit pathogenen Keimen zu schützen, Als Konservierungsstoffe eignen sich die dem Stand der Technik bekannten, insbesondere Benzalkoniumchlorid oder Benzoesäure bzw. Benzoate wie Natriumbenzoat in der aus dem Stand der Technik bekannten Konzentration. Bevorzugt wird der erfindungsgemäßen Formulierung Benzalkoniumchlorid beigemischt. Die Menge des Benzalkoniumchlorids beträgt dabei zwischen 5 mg / 100 ml Formulierung und 20 mg / 100 ml Formulierung, bevorzugt zwischen 5 mg / 100 ml Formulierung und 15 mg / 100 ml Formulierung, stärker bevorzugt zwischen 8 mg / 100 ml Formulierung und 12 mg / 100 ml Formulierung, am stärksten bevorzugt bei 10 mg / 100 ml Formulierung.

Bevorzugte Formulierungen enthalten außer dem Lösungsmittel Wasser und dem Tiotropiumsalz nur noch Benzalkoniumchlorid, Natriumedetat und die zum Einstellen des pH-Werts notwendige Säure, bevorzugt Salzsäure.

Wie bereits erwähnt, wird Tiotropiumbromid in der EP 418 716 A1 oder US 5,610,163 beschrieben.

Kristallines Tiotropiumbromid-Monohydrat kann ebenfalls mittels aus dem Stand der Technik bekannter Herstellverfahrens erhalten werden (WO 02/30928).

Die erfindungs gemäßen Arzneimittelzubereitungen 2 mit Tiotropiumsalzen werden in dem Inhalator der vorstehend beschriebenen Art verwendet, um daraus die erfindungsgemäßen treibgasfreien Aerosole herzustellen.

Wird die erfindungsgemäße Formulierung mittels des vorstehend beschriebenen Zerstäubers 1 vernebelt, sollte die ausgebrachte Masse bei wenigstens 97%, bevorzugt wenigstens 98% aller Betätigungen des Inhalators (Hub oder Hübe) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

### Beispiele

100 ml der Arzneimittelzubereitung 2 enthalten:

| Beispiele | Menge an Tiotropium-bromid mit einem Anteil bezogen auf Tiotropium; | Menge an Tiotropium-bromid- Monohydrat | Menge an Benzalkonium-chlorid | Menge an Natriumedetat | pH-Wert, eingestellt mit HCl (1N) |
|---|---|---|---|---|---|
| 1 | 0,045 g | --- | 10 mg | 10 mg | 2,9 |
| 2 | | 0,057 | 10 mg | 10 mg | 2,9 |
| 3 | 0,023 g | --- | 10 mg | 10 mg | 2,9 |
| 4 | | 0,028 | 10 mg | 10 mg | 2,9 |
| 5 | 0,045 g | --- | 10 mg | 10 mg | 2,8 |
| 6 | | 0,057 | 10 mg | 10 mg | 2,8 |
| 7 | 0,023 g | --- | 10 mg | 10 mg | 2,8 |
| 8 | | 0,028 | 10 mg | 10 mg | 2,8 |
| 9 | 0,045 g | --- | 10 mg | 10 mg | 3,0 |
| 10 | | 0,057 | 10 mg | 10 mg | 3,0 |
| 11 | 0,023 g | --- | 10 mg | 10 mg | 3,0 |
| 12 | | 0,028 | 10 mg | 10 mg | 3,0 |

Der restliche Bestandteil ist geeinigtes Wasser bzw. Wasser für Injektionszwecke mit einer Dichte von 1,00 g/cm³ bei einer Temperatur von 15°C bis 31°C.

Weitere Beispiele 13 bis 24: Analog Beispiele 1 bis 12, aber mit 9 mg Natriumedetat.

Weitere Beispiele 25 bis 36: Analog Beispiele 1 bis 12, aber mit 11 mg Natriumedetat.

Weitere Beispiele 37 bis 48: Analog Beispiele 1 bis 12, aber mit 9 mg Benzalkoniumchlorid.

Weitere Beispiele 49 bis 60: Analog Beispiele 1 bis 12, aber mit 1 mg Benzalkoniumchlorid.

In weiteren Beispielen beträgt die Menge an Benzalkoniumchlorid 8 bzw. 12 mg.

In weiteren Beispielen beträgt die Menge an Natriumedetat 8 bzw. 12 mg.

Unter den Beispielen sind die Beispiele 1 bis 4 am stärksten bevorzugt.

Die Herstellung der vorstehend genannten Formulierungsbeispiele kann ausgehend von kristallinem Tiotropiumbromid-Monohydrat durch Lösen der jeweiligen Formulierungsbestandteile in an sich bekannter Art und Weise erfolgen (WO 02/36104, WO 03/084519).

### Bezugszeichenliste

- 1: Zerstäuber
- 2: Arzneimittelzubereitung
- 3: Behälter
- 4: Beutel
- 5: Druckerzeuger 40
- 6: Halterung
- 7: Antriebsfeder
- 8: Sperrelement
- 9: Förderrohr
- 10: Rückschlagventil 45
- 11: Druckkammer
- 12: Austragsdüse
- 12a: Platte
- 12b: Platte
- 12c: Strömungsbereich 50
- 12d: Auslaßkanal
- 12e: Düsenöffnungen
- 13: Mundstück
- 14: Aerosol
- 15: Zuluftöffnung 55
- 16: erstes Gehäuseteil (Oberteil)
- 16a: Zahn
- 17: Innenteil
- 17a: oberer Teil des Innenteils
- 17b: unterer Teil des Innenteils
- 18: zweites Gehäuseteil (Unterteil)
- 19: Halteelement
- 20: Feder (im Gehäuseunterteil)
- 21: Behälterboden
- 22: Anstechelement
- 23: Zentralteil
- 24: Dichtung
- 25: Stützring
- 26: Überwurfmutter
- 27: Vorfilter
- 28: Filterhalter
- 29: Dichtung
- 30: Düsendichtung
- 31: Halteelement
- 32: Halteteil
- 33: Überwurfmutter
- 34: Durchgangsloch
- 35: Ventilkörper
- 36: Ringschalter
- 37: Verschlußring
- 38: Abgleitfläche
- 39: Sperring
- 40: Taste
- 41: Zähleinrichtung
- 42: Gewindespindel
- 42a: Abschnitt
- 43: Reiter
- 44: Zählmarkierung
- 45: Betätigungsarm
- 46: Sperrfeder
- 47: Ausnehmung
- 48: Mündstückabdeckung

## Patentansprüche

1. Zerstäuber (1) zur Zerstäubung einer Arzneimittelzubereitung (2), mit einer Austragsdüse (12) zur Ausgabe der Arzneimittelzubereitung (2) insbesondere in die Umgebung und/oder ein Mundstück (13),
**dadurch gekennzeichnet,**
**daß** der Zerstäuber (1) einen Vorfilter (27) aufweist, der stromauf der Austragsdüse (12) angeordnet ist, und/oder
**daß** der Zerstäuber (1) ein Halteelement (31) aufweist, das stromab der Austragsdüse (12) angeordnet ist und diese hält.

2. Zerstäuber nach Anspruch 1, **dadurch gekennzeichnet, daß** der Vorfilter (27) unmittelbar mit der Austragsdüse (12) zusammengebaut ist.

3. Zerstäuber nach Anspruch 1 oder 2, **dadurch gekennzeichnet, daß** der Vorfilter (27) und die Austragsdüse (12) von einem gemeinsamen Düsenhalter, insbesondere einer Überwurfmutter (33), gehalten sind.

4. Zerstäuber nach einem der voranstehenden Ansprüche, **dadurch gekennzeichnet, daß** der Vorfilter (27) aus gesintertem Kunststoff hergestellt ist.

5. Zerstäuber nach einem der voranstehenden Ansprüche, **dadurch gekennzeichnet, daß** der Vorfilter (27) Teilchen von etwa 10 µm oder mehr herausfiltert.

6. Zerstäuber nach einem der voranstehenden Ansprüche, **dadurch gekennzeichnet, daß** der Zerstäuber (1) einen vorzugsweise von der Austragsdüse (12) gebildeten Feinstfilter aufweist, wobei der Vorfilter (27) stromauf des Feinstfilters angeordnet ist.

7. Zerstäuber nach einem der voranstehenden Ansprüche, **dadurch gekennzeichnet, daß** das Halteelement (31) scheibenartig oder folienartig ausgebildet ist.

8. Zerstäuber nach einem der voranstehenden Ansprüche, **dadurch gekennzeichnet, daß** das Halteelement (31) ein Durchgangsloch (34) zum Durchtritt der von der Austragsdüse (12) abgegebenen Arzneimittelformulierung (2) aufweist.

9. Zerstäuber nach einem der voranstehenden Ansprüche, **dadurch gekennzeichnet, daß** das Halteelement (31) weicher als die Austragsdüse (12) ausgebildet ist und/oder eine sich seitlich an die Austragsdüse (12) anschließende Düsendichtung (30) zumindest teilweise überdeckt.

10. Zerstäuber nach einem der voranstehenden Ansprüche, **dadurch gekennzeichnet, daß** das Halteelement (31) gegen die Austragsdüse (12) gepreßt ist.

11. Zerstäuber nach einem der voranstehenden Ansprüche, **dadurch gekennzeichnet, daß** die Austragsdüse (12) zwei zueinander geneigte Auslaßkanäle (12d) aufweist, so daß daraus austretende Strahlen der Arzneimittelformulierung (2) sich kreuzen.

12. Zerstäuber (1) zur Zerstäubung einer Arzneimittelzubereitung (2), insbesondere nach einem der voranstehenden Ansprüche, mit einer Zähleinrichtung (41) zur Zählung von Betätigungen des Zerstäubers (1), insbesondere wobei die Zähleinrichtung (41) derart ausgebildet ist, daß der Zerstäuber (1) gegen ein erneutes Betätigen gesperrt wird, wenn eine bestimmte Anzahl von Betätigungen des Zerstäubers (1) erreicht oder überschritten wird (Endsperrzustand),
**dadurch gekennzeichnet,**
**daß** der Zerstäuber (1) ein erstes Gehäuseteil (16) und ein zum Betätigen oder Spannen des Zerstäubers (1) relativ zum ersten Gehäuseteil (16) drehbares Teil (17) aufweist, wobei die Relativdrehung die Zähleinrichtung (41) antreibt, und/oder
**daß** die Zähleinrichtung (41) eine Gewindespindel (42) mit einem zugeordneten, durch Drehen der Gewindespindel (42) insbesondere linear bewegbaren Reiter (43) sowie dem Reiter (43) zugeordnete Zählmarkierungen (44) aufweist.

13. Zerstäuber (1) zur Zerstäubung einer Arzneimitteizubereitung (2), insbesondere nach einem der voranstehenden Ansprüche, mit einer Zähleinrichtung (41) zur Zählung von Betätigungen des Zerstäubers (1), insbesondere wobei die Zähleinrichtung (41) derart ausgebildet ist, daß der Zerstäuber (1) gegen ein erneutes Betätigen gesperrt wird, wenn eine bestimmte Anzahl von Betätigungen des Zerstäubers (1) erreicht oder überschritten wird (Endsperrzustand),
**dadurch gekennzeichnet,**
**daß** der Zerstäuber (1) derart ausgebildet ist, daß der Zerstäuber (1) zur Ausbringung einer Dosis der Arzneimittelzubereitung (2) zweimal hintereinander betätigt, nämlich zweimal hintereinander gespannt und ausgelöst, werden muß und diese zweimalige Betätigung nur als eine Betätigung oder abgegebene Dosis des Zerstäubers (1) von der Zähleinrichtung (41) erfaßt oder gezählt wird, und/oder
**daß** der Zerstäuber (1) ein erstes Gehäuseteil (16) und ein zum Spannen des Zerstäubers (1) relativ zum ersten Gehäuseteil (16) um jeweils 180° drehbares Teil (17) aufweist, wobei diese Relativdrehung um 180° jeweils eine Gewindespindel (42) der Zähleinrichtung (41) weiterdreht, insbesondere jeweils um eine halbe Umdrehung.

14. Zerstäuber nach Anspruch 12 oder 13, **dadurch gekennzeichnet, daß** der Reiter (43) eine Blockiereinrichtung, insbesondere eine Sperrfeder (46), beim oder zum Erreichen des Endsperrzustands betätigt, insbesondere axial verschiebt, um ein radiales Ausfedern zu ermöglichen und/oder eine radiale Verblockung bzw. Sperrung zu erreichen, so daß ein Drehen oder Weiterdrehen des Teils (17) relativ zum Zerstäuber (1) blockiert wird.

15. Zerstäuber (1) zur Zerstäubung einer Arzneimittelzubereitung (2), insbesondere nach einem der voranstehenden Ansprüche, mit einer Zähleinrichtung (41) zur Zählung von Betätigungen des Zerstäubers (1), insbesondere wobei die Zähleinrichtung (41) derart ausgebildet ist, daß der Zerstäuber (1) gegen ein erneutes Betätigen gesperrt wird, wenn eine bestimmte Anzahl von Betätigungen des Zerstäubers (1) erreicht oder überschritten wird (Endsperrzustand),
**dadurch gekennzeichnet,**
**daß** der Zerstäuber (1) ein erstes Gehäuseteil (16) und ein zum Spannen des Zerstäubers (1) relativ zum ersten Gehäuseteil (16) drehbares Teil (17) aufweist, wobei diese Relativdrehung die Zähleinrichtung (41) antreibt,
**daß** der Zerstäuber (1) derart ausgebildet ist, daß der Zerstäuber (1) zur Ausbringung einer Dosis der Arzneimittelzubereitung (2) zweimal hintereinander betätigt, nämlich zweimal hintereinander gespannt und ausgelöst, werden muß und diese zweimalige Betätigung nur als eine Betätigung oder abgegebene Dosis des Zerstäubers (1) von der Zähleinrichtung (41) gezählt wird,
**daß** die Zähleinrichtung (41) eine Gewindespindel (42) mit einem zugeordneten, durch Drehen der Gewindespindel (42) linear bewegbaren Reiter (43) sowie dem Reiter (43) zugeordnete Zählmarkierungen (44) aufweist, und
**daß** der Reiter (43) eine Sperrfeder (46) beim oder zum Erreichen des Endsperrzustands axial verschiebt, um ein radiales Ausfedern zu ermöglichen und/oder eine radiale Verblockung bzw. Sperrung zu erreichen, so daß ein Weiterdrehen des Teils (17) relativ zum Zerstäuber (1) blockiert wird.

16. Zerstäuber nach einem der Ansprüche 12 bis 15, **dadurch gekennzeichnet, daß** der Zerstäuber einen Druckerzeuger (5) und/oder eine Antriebsfeder (7) aufweist, wobei ein Spannen des Druckerzeugers (5) bzw. der Antriebsfeder (7) im Endsperrzustand gesperrt ist und/oder die Zähleinrichtung (41) jedes Spannen der Antriebsfeder (7) oder des Druckerzeugers (5) erfaßt und als Betätigung des Zerstäubers (1) zählt.

17. Zerstäuber nach einem der Ansprüche 12 bis 16, **dadurch gekennzeichnet, daß** das Teil (17) im Endsperrzustand gegen Drehen gesperrt ist und/oder daß die Zähleinrichtung (41) an dem drehbaren Teil (17), insbesondere einem Innenteil (17), im Zerstäuber (1) angeordnet ist.

18. Zerstäuber nach einem der Ansprüche 12 bis 17, **dadurch gekennzeichnet, daß** der Zerstäuber (1) ein zweites Gehäuseteil (18) aufweist, das mit dem Teil (17) insbesondere durch Aufstecken drehgekoppelt ist, so daß durch manuelles Drehen des zweiten Gehäuseteils (18) das Teil (17) relativ zum ersten Gehäuseteil (16) drehbar ist, sofern kein Endsperrzustand vorliegt.

19. Zerstäuber nach einem der Ansprüche 12 bis 18, **dadurch gekennzeichnet, daß** das erste Gehäuseteil (16) einen Zahn oder mehrere Zähne (16a), insbesondere vier Zähne (16a), zum Antrieb der Gewindespindel (42) aufweist.

20. Zerstäuber nach einem der Ansprüche 12 bis 19, **dadurch gekennzeichnet, daß** die Gewindespindel (42) mit dem Reiter (43) ein selbstsperrendes oder selbsthemmendes Schneckengetriebe bildet.

21. Zerstäuber nach einem der Ansprüche 12 bis 20, **dadurch gekennzeichnet, daß** die Zähleinrichtung (41) bzw. die Zählmarkierungen (44) noch ausgebbare Dosen der Arzneimittelformulierung (2) anzeigt bzw, anzeigen.

22. Zerstäuber nach einem der Ansprüche 12 bis 21, **dadurch gekennzeichnet, daß** die Zähleinrichtung (41) und insbesondere der Zerstäuber (1) nur mechanisch arbeiten.

23. Zerstäuber (1) zur Zerstäubung einer Arzneimittelzubereitung (2), insbesondere nach einem der voranstehenden Ansprüche, mit einem Behälter (3) mit der Arzneimittelzubereitung (2),
**dadurch gekennzeichnet,**
**daß** die Arzneimittelzubereitung (2) ein oder mehrere Tiotropiumsalz(e) als Wirkstoff enthält.

24. Zerstäuber nach einem der voranstehenden Ansprüche, **dadurch gekennzeichnet, daß** die Arzneimittelzubereitung (2) enthält oder besteht aus einem oder mehreren Tiotropiuoxasalz(en) als Wirkstoff, in einer Konzentration bezogen auf Tiotropium zwischen 0,01 g pro 100 ml Formulierung und 0,06 g pro 100 ml Formulierung, wobei das (die) Tiotropiumsalz(e) in der Arzneimittelzubereitung vollständig gelöst vorliegt bzw. vorliegen,
Wasser als alleinigem Lösungsmittel,
Säure zum Einstellen eines pH-Werts zwischen 2,7 und 3,1 bevorzugt 2,8 und 3,05,
einem pharmakologisch verträglichen Konservierungsmittel, und einem pharmakologisch akzeptablen Komplexbildner und/oder Stabilisator und/oder optional einem oder mehreren anderen pharmakologisch akzeptablen Hilfs- und Zusatzstoff(en).

25. Zerstäuber (1) zur Zerstäubung einer Arzneimittelzubereitung (2), insbesondere nach einem der voranstehenden Ansprüche, mit einer Zähleinrichtung (41) zur Zählung von Betätigungen des Zerstäubers (1), insbesondere wobei die Zähleinrichtung (41) derart ausgebildet ist, daß der Zerstäuber (1) gegen ein erneutes Betätigen gesperrt wird, wenn eine bestimmte Anzahl von Betätigungen des Zerstäubers (1) erreicht oder überschritten wird (Endsperrzustand),
**dadurch gekennzeichnet,**
**daß** der Zerstäuber (1) derart ausgebildet ist, daß der Zerstäuber (1) zur Ausbringung einer Dosis der Arzneimittelzubereitung (2) zweimal hintereinander betätigt, nämlich zweimal hintereinander gespannt und ausgelöst, werden muß und diese zweimalige Betätigung nur als eine Betätigung oder abgegebene Dosis des Zerstäubers (1) von der Zähleinrichtung (41) erfaßt oder gezählt wird, und
**daß** die Arzneimittelzubereitung (2) enthält oder besteht aus einem oder mehreren Tiotropiumsalz(en) als Wirkstoff, in einer Konzentration bezogen auf Tiotropium zwischen 0,01 g pro 100 ml Formulierung und 0,06 g pro 100 ml Formulierung, wobei das (die) Tiotropiumsalz(e) in der Arzneimittelzubereitung vollständig gelöst vorliegt bzw. vorliegen,
Wasser als alleinigem Lösungsmittel,
Säure zum Einstellen eines pH-Werts zwischen 2,7 und 3,1 bevorzugt 2,8 und 3,05,
einem pharmakologisch verträglichen Konservierungsmittel, und einem pharmakologisch akzeptablen Komplexbildner und/oder Stabilisator und/oder optional einem oder mehreren anderen pharmakologisch akzeptablen Hilfs- und Zusatzstoff(en).

26. Zerstäuber nach einem der Ansprüche 22 bis 25, **dadurch gekennzeichnet, daß** das Tiotropiumsalz ein Salz ausgewählt aus der Gruppe bestehend aus Bromid, Chlorid, Iodid, Monomethylsulfat, Methansulfonat und p-Toluolsulfonat ist.

27. Zerstäuber nach einem der Ansprüche 22 bis 26, **dadurch gekennzeichnet, daß** der Wirkstoff nur Tiotropiumbromid ist.

28. Zerstäuber nach einem der Ansprüche 22 bis 27, **dadurch gekennzeichnet, daß** die Konzentration bezogen auf Tiotropium zwischen 0,02 g /100 ml Arzneimittelzubereitung (2) bis 0,05 g / 100 ml Arzneimittelzubereitung (2), bevorzugt zwischen 0,023 + 0,001g pro 100 ml Arzneimittelzubereitung (2) bis zu 0,045 + 0,001g pro 100 ml Arzneimittelzubereitung (2) beträgt.

29. Zerstäuber nach einem der Ansprüche 22 bis 28, **dadurch gekennzeichnet, daß** die Arzneimittelzubereitung (2) neben Wasser, dem Tiotropiumsalz, Benzalkoniumchlorid, Natriumedetat, Salzsäure und gegebenenfalls Natriumchlorid keine weiteren Hilfs- und Zusatzstoffe enthält.

30. Zerstäuber nach einem der Ansprüche 22 bis 29, **dadurch gekennzeichnet, daß** 100 ml der Arzneimittelzubereitung (2) hergestellt werden durch Lösen von 0,057 g Tiotropiumbromid-Monohydrat, 10 mg wasserfreiem Benzalkoniumchlorid, 10 mg Natriumedetat in Wasser ad 100 ml und 1N Salzsäure zum Einstellen eines pH-Werts von 2,9.

31. Zerstäuber nach einem der Ansprüche 22 bis 29, **dadurch gekennzeichnet, daß** 100 ml der Arzneimittelzubereitung (2) hergestellt werden durch Lösen von 0,028 g Tiotropiumbromid-Monohydrat, 10 mg wasserfreiem Benzalkoniumchlorid, 10 mg Natriumedetat in Wasser ad 100 ml und 1N Salzsäure zum Einstellen eines pH-Werts von 2,9.

32. Zerstäuber nach einem der Ansprüche 22 bis 29, **dadurch gekennzeichnet, daß** 100 ml der Arzneimittelzubereitung (2) hergestellt werden durch Lösen des Tiotropiumsalzes in einer Menge von 0,045 g bezogen auf Tiotropium, 10 mg wasserfreiem Benzalkoniumchlorid, 10 mg Natriumedetat in Wasser ad 100 ml und 1N Salzsäure zum Einstellen eines pH-Werts von 2,9.

33. Zerstäuber nach einem der Ansprüche 22 bis 29, **dadurch gekennzeichnet, daß** 100 ml der Arzneimittelzubereitung (2) hergestellt werden durch Lösen des Tiotropiumsalzes in einer Menge von 0,023 g bezogen auf Tiotropium, 10 mg wasserfreiem Benzalkoniumchlorid, 10 mg Natriumedetat in Wasser ad 100 ml und 1N Salzsäure zum Einstellen eines pH-Werts von 2,9.

34. Zerstäuber nach einem der voranstehenden Ansprüche, **dadurch gekennzeichnet, daß** die Arzneimittelzubereitung (2) als Komplexbildner Etidinsäure oder ein pharmakologisch verträgliches Salz davon enthält.

35. Zerstäuber nach einem der voranstehenden Ansprüche, **dadurch gekennzeichnet, daß** die Arzneimittelzubereitung (2) als Komplexbildner Natriumedetat enthält.

36. Zerstäuber nach Anspruch 35, **dadurch gekennzeichnet, daß** Natriumedetat in einer Menge von 5 mg / 100 ml Arzneimittelzubereitung (2) bis 20 mg / 100 ml Arzneimittelzubereitung (2) enthalten ist, bevorzugt zwischen 8 mg / 100 ml Arzneimittelzubereitung (2) und 12 mg / 100 ml Arzneimittelzubereitung (2).

37. Zerstäuber nach einem der voranstehenden Ansprüche, **dadurch gekennzeichnet, daß** der pH-Wert der Arzneimittelzubereitung (2) zwischen 2,8 und 3,0, bevorzugt bei 2,9 liegt.

38. Zerstäuber nach einem der voranstehenden Ansprüche, **dadurch gekennzeichnet, daß** der pH-Wert der Arzneimittelzubereitung (2) mit einer anorganischen Säure, bevorzugt Salzsäure eingestellt wird.

39. Zerstäuber nach einem der voranstehenden Ansprüche, **dadurch gekennzeichnet, daß** die Arzneimittelzubereitung (2) als Konservierungsmittel Benzalkoniumchlorid enthält.

40. Zerstäuber nach einem der voranstehenden Ansprüche, **dadurch gekennzeichnet, daß** der Zerstäuber (1) pro Betätigung 10 bis 50 µl der Arzneimittelzubereitung (2) ausgibt und zerstäubt.

41. Zerstäuber nach einem der voranstehenden Ansprüche, **dadurch gekennzeichnet, daß** der Zerstäuber (1) definierte Mengen der Arzneimittelzubereitung (2) unter Drücken von 10 bis 60 MPa zerstäubt.

42. Zerstäuber (1) zur Zerstäubung einer Arzneimittelzubereitung (2), insbesondere nach einem der voranstehenden Ansprüche, mit einem Behälter (3) mit der Arzneimittelzubereitung (2),
**dadurch gekennzeichnet,**
**daß** der Zerstäuber (1) eine Einrichtung zur zwangsweisen Belüftung des Behälters (3) aufweist.

43. Zerstäuber nach Anspruch 42, **dadurch gekennzeichnet, daß** die Einrichtung ein insbesondere von einer Feder (20) gehaltenes oder gebildetes Anstechelement (22) aufweist.

44. Zerstäuber nach Anspruch 42 oder 43, **dadurch gekennzeichnet, daß** der Zerstäuber (1) derart ausgebildet ist, daß der Behälter (3) beim erstmaligen Benutzen des Zerstäubers (1), insbesondere bei axialem Bewegen des Behälters (3) im Zerstäuber (1) und/oder bei Spannen einer Feder (20) des Zerstäubers (1), zur Belüftung zwangsweise geöffnet oder angestochen wird.

45. Zerstäuber nach einem der Ansprüche 42 bis 44, **dadurch gekennzeichnet, daß** der Behälter (3) bodenseitig und/oder an seinem dem Entnahmeende gegenüberliegenden Ende belüftet wird.

46. Zerstäuber nach einem der voranstehenden Ansprüche, **dadurch gekennzeichnet, daß** der Behälter (3) während der Förderung, Druckerzeugung und/oder Zerstäubung vorzugsweise hubartig bewegbar ist.

47. Zerstäuber nach einem der voranstehenden Ansprüche, **dadurch gekennzeichnet, daß** der Zerstäuber (1) ein Förderelement, insbesondere ein Förderrohr (9), zur Förderung der Arzneimittelzubereitung (2) aus dem Behälter (3) aufweist.

48. Zerstäuber nach einem der voranstehenden Ansprüche, **dadurch gekennzeichnet, daß** der Zerstäuber (1) eine Fördereinrichtung, vorzugsweise einen Druckerzeuger (5), insbesondere mit einem Förderelement, wie einem Förderrohr (9), zur Förderung und/oder Zerstäubung der Arzneimittelzubereitung (2) aufweist.

49. Zerstäuber nach Anspruch 47 oder 48, **dadurch gekennzeichnet, daß** der Behälter (3) mittels des Förderelements insbesondere durch Anstechen oder Einführen öffenbar ist.

50. Zerstäuber nach einem der voranstehenden Ansprüche, **dadurch gekennzeichnet, daß** der Zerstäuber (1) derart ausgebildet ist, daß die ausgebrachte Masse der Arzneimittelzubereitung (2) bei wenigstens 97%, insbesondere 98%, aller Betätigungen des Zerstäubers (1) zwischen 5 und 30 mg mit einem Toleranzbereich von 25%, insbesondere 20%, liegt.

51. Zerstäuber nach einem der voranstehenden Ansprüche, **dadurch gekennzeichnet, daß** die Druckerzeugung bzw. Zerstäubung rein mechanisch, insbesondere treibgasfrei, vorzugsweise durch Federkraft, erfolgt.

52. Zerstäuber nach einem der voranstehenden Ansprüche, **dadurch gekennzeichnet, daß** der Zerstäuber (1) einen Druckerzeuger (5) mit einer Antriebsfeder (7) aufweist, insbesondere wobei die Zerstäubung von Fluid (2) ausschließlich durch die Kraft der Antriebsfeder (7) erfolgt.

53. Zerstäuber nach Anspruch 52, **dadurch gekennzeichnet, daß** die Antriebsfeder (7) in dem Zerstäuber (1) vorgespannt eingebaut ist.

54. Zerstäuber nach Anspruch 52 oder 53, **dadurch gekennzeichnet, daß** die Antriebsfeder (7) in einem vorzugsweise drehbaren Teil (17) des Zerstäubers
(1) mittels eines Verschlußrings (37) gesichert oder gehalten ist.

55. Zerstäuber nach einem der voranstehenden Ansprüche, **dadurch gekennzeichnet, daß** der Zerstäuber (1) als vorzugsweise tragbarer Inhalator, insbesondere zur medizinischen Aerosol-Therapie, ausgebildet ist.
